# EUROPEAN PATENT APPLICATION

(11) **EP 1 000 597 A2**
(43) Date of publication of application: **17.05.2000**
(21) Application number: 99308862.4
(22) Date of filing: 08.11.1999
(51) Int. Cl.: A61F 13/15

(54) **Sanitary napkin with rear extension providing a liquid blocking function**

(30) Priority: 09.11.1998 US 189009
(71) Applicant: JOHNSON & JOHNSON INC., Montreal, Quebec H1N 2G4 (CA)
(72) Inventor: Boulanger, Roger, Ste-Julie, Quebec J3E 1C5 (CA)
(74) Representative: Mercer, Christopher Paul

(57) **Abstract**

The present invention is directed to a sanitary napkin having a liquid-absorbing section with two opposing longitudinal sides; a first transverse side which defines a front portion of the liquid-absorbing section and a second transverse side opposing the first transverse side which defines a rear portion of the liquid-absorbing section. The liquid-absorbing section includes a liquid-permeable cover layer, a liquid-impervious barrier layer and an absorbent layer intermediate the cover layer and the barrier layer. The napkin further includes a liquid-arresting section united with the liquid-absorbing section, the liquid-arresting section projecting rearward from the second transverse side of the liquid-absorbing section to a sufficient distance to at least partially cover a wearer's gluteal groove when the liquid-absorbing section is substantially aligned with the wearer's vaginal opening. The liquid-arresting section has an external surface facing away from the wearer's body when the article is in use by the wearer, the external surface being substantially free of adhesive. The liquid-arresting section has a longitudinally extending preferential bending zone, such that when the article is in use by the wearer, at least a portion of the liquid-arresting section is capable of bending at the preferential bending zone so as to enter or conform to the wearer's gluteal groove.

## Description

### FIELD OF THE INVENTION

The present invention relates to an article for absorbing body exudate, more particularly to a sanitary napkin capable of reducing the likelihood of leakage at the rear end, particularly at nighttime when the wearer has adopted a horizontal posture.

### BACKGROUND OF THE INVENTION

Traditional sanitary napkins, when worn during the night are prone to failures in the rear end area, because the menstrual liquid has a tendency to flow under the effect of gravity toward the buttocks. (This problem is less likely to happen during daytime when the wearer is standing up, as in such position, gravity actually causes the menstrual liquid to penetrate through the cover layer of the napkin and migrate toward the absorbent layer where it is permanently retained.) To limit the occurrence of night failures, manufacturers have developed specially designed napkins that offer enhanced absorptive capability near the rear end. More specifically, such napkins are made longer and somewhat wider so as to offer a greater surface area to capture menstrual liquid. The theory behind this approach is that free liquid that may have traveled along the body of the wearer or along the cover layer, to the buttocks area of an article, that would not have been absorbed in an article designed for daytime use, would be absorbed in the rear extension of an article designed for nighttime use. Thus, the chances of failures are reduced.

Although sanitary napkins made for nighttime use are less likely to suffer from rear end failures in comparison with sanitary napkin designs made for daytime use, nighttime failures do still occur. One problem is that exudate to be absorbed may flow along the cover layer of the napkin in the gap created by the gluteal groove of the wearer. Conventional napkins do not address this problem as they have all been designed to lie relatively flat against the body. Furthermore, conventional napkins all have adhesive at the rear extremity of their garment-facing surface. This adhesive, amongst other things, will generally hold the napkin securely to the undergarment and prevent the napkin from conforming to the shape of the buttocks (e.g. gluteal groove) in that region of the body.

Another drawback with conventional sanitary products for nighttime use is their less than ideal comfort potential. In this respect, for at least some consumers, the presence of a large rear extension creates a perception of a product that is excessively bulky, less comfortable, and impractical to install in an undergarment.

Furthermore, from a manufacturing point of view, conventional nighttime-use designs, although effective, are more expensive to produce than daytime-use designs because more raw materials are required to make the rear extension.

In view of the foregoing there is a need in the industry to develop a sanitary napkin offering an enhanced protection against failures at the rear end, particularly when the wearer has adopted a horizontal posture. There is also a need to develop a sanitary napkin designed for nighttime use that is more comfortable to wear and also less costly to manufacture than traditional sanitary napkins designed for nighttime use.

### OBJECT AND STATEMENT OF THE INVENTION

It is therefore an object of the present invention to provide an improved sanitary napkin designed for nighttime use.

It is a further object of the present invention to provide a sanitary napkin designed for nighttime use that is better adapted than conventional designs to conform to the body of the wearer in the rear portion of the napkin, particularly with respect to the gluteal groove.

It is still a further object of the present invention to provide a sanitary napkin designed for nighttime use that makes more efficient use of absorbent material and capacity.

It is yet another object of the present invention to provide a sanitary napkin designed for nighttime use that is relatively less expensive to manufacture than conventional nighttime use napkin designs.

Hence, as embodied and broadly described herein, the present invention provides a sanitary napkin, comprising:
(A) a liquid-absorbing section for absorbing bodily exudate, said liquid-absorbing section having:
   (i) two opposing longitudinal sides;
   (ii) a first transverse side defining a front portion of said liquid-absorbing section;
   (iii) a second transverse side opposing the first transverse side, the second transverse side defining a rear portion of said liquid-absorbing section;
   (iv) an imaginary longitudinal centerline; and
   (v) an imaginary transverse centerline;
      said liquid-absorbing section comprising:
   (i) a liquid-permeable cover layer, the cover layer having an external surface facing towards a wearer's body when the napkin is in use by the wearer,
   (ii) a liquid-impervious barrier layer, the barrier layer having an external surface facing away from the wearer's body when the napkin is in use by the wearer;
   (iii) an absorbent layer intermediate the cover layer and the barrier layer;
   (iv) adhesive material on the external surface of the barrier layer, the adhesive material capable of releasably adhering the liquid-absorbing section to an undergarment of the wearer when the napkin is in use;
(B) a liquid-arresting section united with said liquid-absorbing section, said liquid-arresting section projecting rearward from the second transverse side of said liquid-absorbing section to a sufficient distance to at least partially cover a wearer's gluteal groove when the liquid-absorbing section is substantially aligned with the wearer's vaginal opening, the liquid-arresting section having an external surface facing away from the wearer's body when the article is in use by the wearer, the external surface being substantially free of adhesive material.

### Liquid-Absorbing Section

In the context of the present invention, the liquid-absorbing section is somewhat analogous to the main body of a standard non-nighttime conventional sanitary napkin. It is the part of the napkin that aligns with or covers wholly or in large part the vaginal opening of the wearer. As such, it is preferred that the liquid-absorbing section of the napkin be generally rectangular in shape with rounded ends. When the article is of such a conformation, the first and second transverse sides are the shorter opposing sides and will generally have an arcuate shape. The first transverse side defines a front portion of the liquid-absorbing section, *i.e.* the portion of the liquid-absorbing section which, when the napkin is in use, will normally lie on the front side (anterior side) of the wearer's body. Opposing the first transverse side is the second transverse side. The second transverse side defines a rear portion of the liquid-absorbing section, *i.e.* the portion of the liquid-absorbing section which, when the napkin is in use, will normally lie on the rear side (posterior side) of the wearer's body.

The first and second longitudinal sides are the longer opposing sides and will generally be straight or slightly arcuate. It should be understood however that the shape of such sides is not essential to the invention, and thus the transverse or the longitudinal sides could be either arcuate or straight. Nor is it essential that the liquid-absorbing section be generally rectangular, as articles having a liquid-absorbing section in the shape of a square, parallelogram or any other polygon, or even that of an oval, hour-glass or dog-bone, are all within the scope of the present invention. Where such is the case *(i.e.* where the liquid-absorbing section is not rectangular), the longitudinal sides of the liquid-absorbing section will be considered to be those opposing sides of a greater relative length as compared with the other opposing sides of the main body. Where it is impossible to make such a determination, any opposing pair of sides may be considered to be the longitudinal sides, or at the limit, opposing portions of any two sides may be considered to be the longitudinal sides.

For most napkins, the longitudinal centerline of the napkin is an imaginary line that extends longitudinally along the intermediate portion of the napkin that is equidistant from the longitudinal sides thereof. It will thus bisect the napkin into two generally mirror image halves. Thus, when the napkin is in use by a wearer, the longitudinal centerline thereof is generally parallel to, or lies in, the sagittal plane of the wearer. Similarly, for most napkins, the transverse centerline is an imaginary line that extends transversally across the article, and is typically, but not always, equidistant from the transverse sides thereof. The transverse centerline is thus perpendicular to the longitudinal centerline. Where the article has flaps (as described below), the transverse centerline is the line perpendicular to the longitudinal centerline that bisects the flaps.

The liquid-absorbing section preferably comprises at least three distinct layers united together in a superimposed relationship. It is not, however, limited to napkins having liquid-absorbing sections that only have three layers. There are many additional conventional layers and/or other structures that may also be present, all within the scope of the present invention.

The layer that is normally in contact with the body of the wearer when the napkin is in use is conventionally termed the cover layer or top sheet. The primary purpose of cover layer is permit the rapid ingress of exudate to be absorbed into the napkin, all the while remaining dry and comfortable to the user of the napkin. The cover layer has two major surfaces, an external surface *(i.e.* a surface that does not face another layer of the napkin) that faces the body of the wearer when the napkin is in use, and an internal surface that faces the absorbent layer.

The absorbent layer is below the cover layer. The absorbent layer may comprise a single layer or a composite layer combining multiple layers or additional layers. It is preferably combined with other layers and/or structures, most preferably a transfer layer, to form an entire absorbent system. The primary purpose of all of these structures is to absorb and retain bodily exudate, or to facilitate this process. The absorbent layer has two major surfaces, both of which are internal surfaces, *i.e.* they both face other layers of the napkin. The first surface faces the cover layer, the second surface faces the barrier layer.

Underneath the absorbent layer is the barrier layer or backsheet. The primary purpose of the barrier layer is to prevent exudate absorbed within the napkin from egressing the napkin on the opposite side from which it was absorbed. It is thus highly preferred that the barrier layer be impervious to liquid. It may also be preferred, for some applications, that the barrier layer be impervious to gases as well. The barrier layer has two major surfaces, an external surface that faces the undergarment of the wearer when the napkin is in use, and an internal surface that faces the absorbent layer.

Located on the garment-facing surface of the barrier layer is adhesive material for releasably adhering the liquid-absorbing section to the undergarment of the wearer. In the context of the present specification, it is preferred that the adhesive material employed be capable of firmly affixing the liquid-absorbing section to the undergarment in a manner such that when the napkin is in use, the liquid-absorbing section will not become detached from the undergarment without human intervention. There are many conventional adhesive materials that will suffice to accomplish this function; a simple chemical adhesive is preferred.

The cover layer and the barrier layer are preferably joined to one another around the periphery of the article to form a seal enclosing the absorbent layer, and forming a structurally integral napkin. It is preferred that this seal surround, and thus contain, the absorbent layer within it. All conventional manners of sealing and/or uniting the various layers together are within the scope of the present invention.

### Liquid-Arresting Section

United with, and projecting laterally from the rear of the liquid-absorbing section rearward when the napkin is in a flattened state, is a liquid-arresting portion. No particular shape is essential to the liquid-arresting portion. Typically, the liquid-arresting portion will be a natural extension of the liquid-absorbing section, *i.e*. it will be a continuation of the same overall shape. The liquid-arresting section extends beyond the second transverse side of the liquid-absorbing portion a distance sufficient to at least partially cover the wearer's gluteal groove when the absorbent section is substantially aligned with or covering the wearer's vaginal opening. The exact length of the liquid-arresting section, or the distance that it extends beyond the second transverse side of the liquid-absorbing section, will depend upon the length of the liquid-absorbing section and the size of the wearer. For example, when the length of the absorbent section is about 24 centimeters, the length of the liquid-arresting section is at least 2.5 centimeters, preferably at least 3.75 centimeters, and more preferably at least 5.0 centimeters, and most preferably at least 7.0 centimeters. The length of extension of the liquid-arresting portion is measured along the line co-linear with the longitudinal centerline of the napkin, from the second transverse side of the liquid-absorbing section to the physical rear edge (the transverse end) of the liquid-arresting section. Furthermore, while not essential, it is preferred, that the liquid-arresting section have a width (*i.e.* its measurement taken in a direction perpendicular to its length) of at least 7.0 cm, in order to cover a significant portion of the wearer's gluteal groove.

The liquid-arresting section may comprise a single layer of material or a composite laminate structure. In either case it is united with the liquid-absorbing section, preferably in one of two ways. In one execution, the liquid-arresting section is not continuous with any of the component layers of the liquid-absorbing section. Rather, the liquid-arresting section is manufactured separately from the liquid-absorbing section and later united therewith in such a manner so as to extend rearward from the liquid absorbing section as a separate entity. In such cases, the second transverse side of the liquid-absorbing section is a physical edge of that section. Typically, the liquid arresting section is joined with the liquid-absorbing section in an overlapping relationship. At the zone of overlap, the two sections are united to one another by any conventional means, *e.g.* adhesive bonding, thermal bonding, etc.

By contrast, in the second, and more preferred, execution, the liquid-arresting section is continuous with, and is an integral extension of, one or more of the component layers of the liquid-absorbing section. The liquid-arresting section might simply be continuous with either the barrier layer of the liquid-absorbing section, the cover layer of the liquid-absorbing section, or preferably both. Additional materials continuous or discontinuous with those of the liquid-absorbing layer may also be present.

In either embodiment, the liquid-arresting portion will have two external surfaces, a first external surface facing the body of the wearer (specifically at least a portion of which will face the wearer's buttocks and gluteal groove) when the napkin is in use. It will also have a second external surface facing the wearer's undergarment

The external surface of the liquid-arresting portion that faces the undergarment of the wearer is substantially free of adhesive material. Preferably, there is a complete absence of adhesive material on this surface. The absence of adhesive material will allow the liquid-arresting portion to conform better to the buttocks of the wearer since it will not be maintained adhered to the wearer's undergarment. Because of natural moisture in this area of the body, it is likely that, in the absence of being adhered to the wearer's undergarment, the liquid-arresting portion will naturally slightly adhere to the skin of the wearer, likely causing that portion to assume the contour of the body in that area, reducing the likelihood of exudate leakage from along the gluteal groove.

To assist in this function, *i.e.* the assumption of the liquid-arresting section of the contour of the buttocks, including the gluteal groove, the section is preferably provided with a longitudinally extending preferential bending zone. By "longitudinally" it is meant that the zone extends in a direction parallel to, and most preferably encompasses, the line co-linear with the longitudinal centerline of the liquid-absorbing section. When located in this region, the zone will, in most wearers of the napkin, register with the gluteal groove of the wearer. By "preferential bending zone" it is meant that the liquid-arresting section will preferentially bend within this zone before bending in other areas. The preferential bending zone is preferably constructed, if possible, such that the section preferentially bends inward towards the body of the wearer (in contrast to bending outward, away from the body of the wearer). In this manner, since the liquid-arresting section is free of adhesive, and is thus free to separate from the undergarment, when it does so, it will preferentially penetrate within the gluteal groove. Hence, the section will better conform to the body of the wearer in this region, even further reducing the risk of failure.

The longitudinally extending preferential bending zone may be formed in a variety of conventional manners. Preferably the materials that comprise the liquid-arresting section are mechanically scored in the region corresponding to the bending zone.

Most preferably, the liquid-arresting section of the sanitary napkin is devoid of any absorbent system. Thus, any absorption capacity available in the liquid-arresting section is present owing to the small absorption capacity of the cover layer part and also the thin void volume that may exist between the cover layer part and the barrier layer part, and that is capable of accepting and retaining liquid. In practice, however, the absorbent capacity of the liquid-arresting section is small by comparison of the absorption capacity of the liquid-absorbing section.

In a possible variant the liquid-arresting section can be provided with an augmented absorption capacity. Where this is the case, it is highly preferred that the average absorption capacity of the liquid-arresting section be less than the average absorption capacity of the liquid-absorption section. The reduction of the absorbent capacity in the liquid-arresting section will typically translate into a reduction in manufacturing expense. Therefore only the minimum amount of absorbent capacity as is required for the particular application should be present. Ratios of 2:1, 3:1, and 5:1 (liquid-absorption section capacity to liquid-arresting section absorbent capacity) are increasingly preferred.

Providing the liquid-arresting section with augmented absorbent capacity can be achieved by extending into that section one of the component layers of the absorbent system of the liquid-arresting section. For example, if the absorbent system includes a transfer layer and an absorbent layer, the transfer layer may extend into the liquid-arresting section. The degree of extension can vary in accordance with the intended application. One possibility is to make the transfer layer portion that enters the liquid-arresting section substantially coextensive with the cover layer part and the barrier layer part. Another possibility is to extend the transfer layer only partially into the liquid-arresting section.

In a further variant, an absorption capacity gradient can be created along the sanitary napkin by providing the liquid-arresting section with an absorbent system whose absorption capacity diminishes progressively toward the rear end of the sanitary napkin. In practice, this can be achieved by producing the absorbent system of the sanitary napkin such that the part contained in the liquid-arresting section has a progressively diminishing thickness (caliper).

Contrary to what conventional wisdom dictates, such reduction (or completely absence) in absorbent capacity of the liquid-arresting section does not create any significant increase in rear failures. This is due to the fact that only a small fraction of the total liquid discharge reaches that liquid-arresting section and a small absorption capacity suffices. In instances where the liquid-arresting section has virtually no absorption capacity, the liquid-impervious nature of the material in that section prevents the liquid from contacting the undergarment and thus suffices to avoid a failure event. Although the liquid may locally pool, the small quantity of liquid that is likely to accumulate develops sufficient surface tension with the cover layer so the liquid is retained to the napkin without escaping and soiling the undergarment.

It is also preferred that the flexibility of the liquid-arresting section along a transverse axis be greater than the flexibility of the liquid-absorbing section also measured along a transverse axis. In this manner, the liquid-arresting section will be able to more easily conform to the shape of the body of the wearer in the gluteal region. Typically, a reduction in the absorption capacity of the liquid-arresting section (as compared with the liquid-absorbing section) increases the flexibility of that section, so additionally efforts in this respect may not need to be made. Ratios of 2:1, 3:1, and 5:1 (flexibility of the liquid-arresting section to liquid-absorbing section) are increasingly preferred.

It is further preferred that the average caliper of the liquid-arresting section be less than the average caliper of the liquid-absorbing section. The reduced caliper, along with the increased flexibility, will generally enhance the comfort of the liquid-arresting section as compared with the rear sections of prior art sanitary napkins designed for nighttime use. Ratios of 2:1, 3:1, and 5:1 (liquid-absorption section caliper to liquid-arresting section caliper) are increasingly preferred.

Moreover, the present inventor has also discovered that an increase in the flexibility and thus conformability of the liquid-arresting section allows that section to behave as a positioning system by enhancing the stability of the sanitary napkin against the perineal area of the user. As mentioned earlier, an increase in flexibility of the liquid-arresting section enables that portion of the sanitary napkin to better adapt and follow the anatomy of the wearer in the buttocks area. Such augmented adaptation results in a greater contact area sanitary napkin/skin with the result that the napkin is less likely to shift in position, and to better maintain a fluid barrier.

### Flaps

Preferably, a sanitary napkin of the present invention further comprises a pair of flaps, one flap projecting from each longitudinal side of the liquid-absorbing section, preferably intermediate and spaced from the transverse sides of the section. The flaps are flexible and are capable of being folded about a side edge of the crotch portion of the undergarment of the wearer when the napkin is in use. The flaps may be of any conventional design, although an isosceles trapezoidal shaped flap with the top adjoined to the longitudinal side of the main body and the base at the distal end is preferred. In use, the flaps help to stabilize the napkin within the undergarment and to protect the side edges of the undergarment from becoming soiled.

The flaps preferably are continuous integral extensions of the cover layer and barrier layer materials of the liquid-absorbing section sealed together. They may however comprise separately formed material attached to the liquid-absorbing section after the manufacture thereof. They may also be formed from a panel projecting outward from the longitudinal side of the liquid-absorbing section that has been attached to the garment-facing surface thereof, as described in commonly-assigned co-pending International Patent Application published as WO 98/27903 in the name of Boulanger *et al.*

Other objects and features of the invention will become apparent by reference to the following specification and to the drawings.

### BRIEF DESCRIPTION OF THE DRAWINGS

The following is a description by way of a preferred embodiment, reference being made to the following drawings, in which:
FIG. 1 is a top plan view of a sanitary napkin constructed in accordance with the present invention, showing the cover layer partially peeled off so as to reveal the transfer layer, the absorbent layer, and the barrier layer;
FIG. 2 is a perspective view of the sanitary napkin of the present invention in a curved conformation, typical of what would be attained when the napkin is placed in the undergarment of the wearer;
FIG 3 is a bottom plan view of the sanitary napkin in Figure 1;
FIG. 4 is a sectional view taken along line 4-4 in Figure 1;
FIG. 5 is a sectional view of an alternate embodiment featuring a liquid-arresting section in which extends the transfer layer of the liquid-absorbing section;
FIG. 6 is a sectional view of an alternate embodiment featuring a single layer absorbent system having a tail element that extends into the liquid-arresting section;
FIG. 7 is a side view of the sanitary napkin in accordance with the present invention shown secured to the undergarment of the wearer (the undergarment is illustrated in broken lines);
FIG. 8 is top plan view of another embodiment of the present invention in which the liquid-arresting section is discontinuous with the liquid-absorbing section;
FIG. 9 is a sectional view taken along line 9-9 in Figure 8; and
FIG. 10 is a sectional view taken along line 10-10 in Figure 8.

In the drawings, preferred embodiments of the invention are illustrated by way of example. It is to be expressly understood that the description and drawings are only for purposes of illustration and as an aid to understanding, and are not intended to be a definition of the limits of the invention.

### DESCRIPTION OF A PREFERRED EMBODIMENT

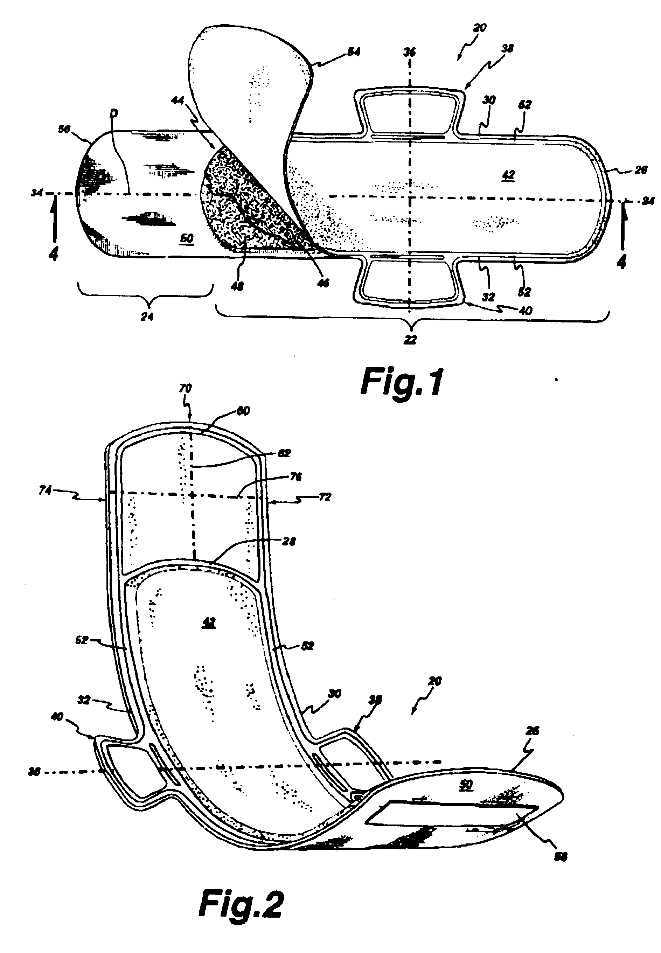

Referring to Figures 1 and 2, there is shown the preferred embodiment of the present invention, a feminine sanitary napkin 20. The napkin 20 comprises two sections, a liquid-absorbing section 22 and a liquid-arresting section 24.

### The Liquid-Absorbing Section

The liquid-absorbing section 22 has a first transverse side 26 defining a front portion thereof and a second transverse side 28 defining a rear portion thereof. Each of these sides are concavely arcuate. The first transverse side 26 is a physical edge of the napkin 20 and the liquid-absorbing section 22; the second transverse side 28 is not. In the present embodiment, the second transverse side 28 is considered to be the peripheral seal 52.

The liquid-absorbing section 22 also has a first longitudinal side 30 and a second longitudinal side 32, each longitudinal side being substantially straight. An imaginary longitudinal centerline 34 runs down the central portion of the liquid-absorbing section 22, equidistant from the longitudinal sides 30, 32.

Projecting laterally outward from each of the longitudinal sides 30, 32 is a flap 38, 40 (respectively). The flaps 38, 40 are in the shape of an isosceles trapezoid with the top adjoining the longitudinal side and the base at the distal end. The liquid-absorbing section 22 also has an imaginary transverse centerline 36 perpendicular to the longitudinal centerline 34 and simultaneously bisecting the flaps 38, 40.

The liquid-absorbing section 22 is of a laminate construction and preferably comprises a fluid-permeable cover layer 42, an absorbent system 44, and a fluid-impervious barrier layer 50. Each of these layers is described in detail hereinbelow.

### Cover Layer

The cover layer 42 may be a relatively low density, bulky, high-loft non-woven web material. The cover layer 42 may be composed of only one type of fiber, such as polyester or polypropylene or it may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. An example is the non-woven cover layer supplied by STEARNS of sanitary napkins sold by Johnson & Johnson Inc. of Montreal, Canada under the trademark Stayfree Ultra-Thin Cottony Dry Cover.

Bi-component fibers may be made up of a polyester layer and a polyethylene sheath. The use of appropriate bi-component materials results in a fusible non-woven fabric. Examples of such fusible fabrics are described in U.S. Patent 4,555,446 issued November 50, 1985 to Mays. Using a fusible fabric increases the ease with which the cover layer may be mounted to the adjacent transfer layer and/or to the barrier layer.

The cover layer 42 preferably has a relatively high degree of wettability, although the polymers comprising the individual fibers comprising the cover may not be particularly hydrophilic. The cover material should also contain a great number of relatively large pores. This is because the cover layer 42 is intended to absorb body fluid rapidly and transport it away from the body and the point of deposition. Preferably, the fibers which make up the cover layer 42 should not lose their physical properties when they are wetted, or in other words, they should not collapse or lose their resiliency when subjected to water or body fluid. The cover layer 42 may be treated to allow fluid to pass through it readily. The cover layer 42 also functions to transfer the fluid quickly to the other layers of the absorbent structure 44. Thus, the cover layer 42 is preferably wettable, hydrophilic and porous. When composed of synthetic hydrophobic fibers such as polyester or bi-component fibers, the cover layer 42 may be treated with a surfactant to impart the desired degree of wettability.

Most preferably, the cover layer 42 is made of polymer film having large pores. Because of such high porosity, the film accomplishes the function of quickly transferring body fluid to the inner layers of the absorbent system. Apertured coextruded films such as RETICULON™ brand, for example, described in U.S. Patent 4,690,679 and available on sanitary napkins sold by Johnson & Johnson Inc. of Montreal, Canada are useful as cover layers in the present invention.

The cover layer 42 may be embossed to the remainder of the absorbent system 44 in order to aid in promoting fluid transport by fusing the cover to the next layer.

### Absorbent System - Transfer Layer

Adjacent to the cover layer 42 on its inner side and bonded to the cover layer 42 is a fluid transfer layer 46 that forms part of the absorbent system 44. The transfer layer 46 provides the means of receiving body fluid from the cover layer 42 and holding it until the highly-dense absorbent layer has an opportunity to absorb the fluid.

The transfer layer 46 is, preferably, more dense than and has a larger proportion of smaller pores than the cover layer 42. These attributes allow the transfer layer 46 to contain body fluid and hold it away from the outer side of the cover layer 42, thereby preventing the fluid from re-wetting the cover layer 42 and its surface. However, the transfer layer 46 is, preferably, not so dense as to prevent the passage of the fluid through the layer 46 into the absorbent layer 48.

The transfer layer 46 may be composed of fibrous materials, such as wood pulp, polyester, rayon, flexible foam, or the like, or combinations thereof. The transfer layer 46 may also comprise thermoplastic fibers for the purpose of stabilizing the layer and maintaining its structural integrity. The transfer layer 46 may be treated with surfactant on one or both sides in order to increase its wettability, although generally the transfer layer 46 is made of relatively hydrophilic materials and may not require treatment. The transfer layer 46 is preferably bonded on both sides to the adjacent layers, i.e. the cover layer 42 and the absorbent layer 48. An example is the material made of non-woven blended wood pulp with a latex binder sold by Buckeye Technologies of Memphis, Tennessee under the designation VICELL 6002.

### Absorbent System - Absorbent Layer

Immediately adjacent to and bonded to the transfer layer 46 is the absorbent layer 48. The absorbent layer 48 is preferably a highly dense layer having a fine porosity. It has a large liquid holding capacity and it is extremely retentive. Most preferably, the absorbent layer 48 is composed of compressed sphagnum moss material. More specifically, the sphagnum moss is formed as a board by air or wet laying and calendering to obtain a relatively thin, i.e. from about 0.025 to 0.25 cm thick, relatively dense, i.e. from about 0.2 to 10 g/cm³ sheet like structure. The structure may include a layer of Kraft tissue laminated on one or both surfaces of the sphagnum moss layer. Preferably, a fibrous component is admixed with the sphagnum moss material. The fibrous component is suitably a natural or synthetic textile fiber such as rayon, polyester, nylon, acrylic or the like, having a length of from about 0.625 to 3.75 cm and a denier of from about 1.0 to 5. The fibrous component may be present in an amount from about 2 to 20% by weight, most preferably from 4 to 8%. The absorbent layer 48 may also comprise other components such as wood pulp, synthetic wood pulp, thermomechanical pulp, mechanically ground pulp, polymers, surfactants, superabsorbents and the like.

In an alternative embodiment the absorbent system 44 includes a single layer of pulp fluff material (the transfer layer is omitted). The absorbent layer 48 preferably comprises a pulp fluff material and includes other absorbent materials or non-absorbent materials which aid in stabilizing the absorbent structure such as conjugate fibers, fusible fibers, binders, sphagnum peat moss particles, superabsorbents, and the like and combinations thereof, and may optionally include other absorbent materials or non-absorbent materials which aid in stabilizing the absorbent structure such as conjugate fibers, fusible fibers, binders, sphagnum peat moss particles, superabsorbents, and the like and combinations thereof.

### Barrier Layer

Underlying the absorbent system 44 is a barrier layer 50 comprising liquid-impervious film material so as to prevent liquid that is entrapped in the absorbent layer 46 from egressing the sanitary napkin and staining the wearer's undergarment. Most preferably, the barrier layer 50 is made of polymeric film, such as polyethylene, which is both inexpensive and readily available. The polyethylene is capable of fully blocking the passage of liquid or gas that may emanate from the absorbent system 44. In a variant, breathable films may be used that allow passage of gases while blocking liquid. A suitable example is a combination polyethylene/ethylene vinyl acetate (EVA) film sold by the Edison Plastics Company in Canada and in the United-States under the designation XP-1167B.

The cover layer 42 and the barrier layer 50 are joined along their marginal portions so as to form an enclosure that maintains the absorbent system 44 captive. The joint may be made by means of adhesives, heat-bonding, ultrasonic bonding, radio frequency sealing, mechanical crimping, and the like and combinations thereof. The peripheral seal line is shown in Figure 1 by the reference numeral 52.

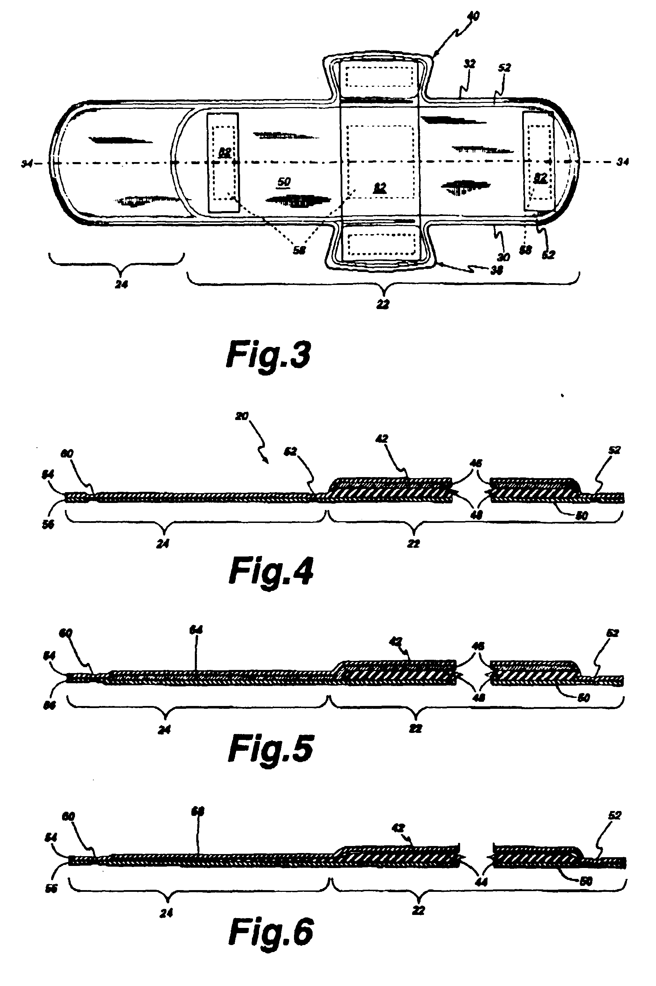

Referring to Figures 2 and 3, in order to enhance the stability of the sanitary napkin, the garment facing surface of the barrier layer 50 of liquid-absorbing section 22 is provided with adhesive material 58, typically hot-melt adhesive material capable of establishing a temporary bond with the undergarment material. A suitable material is the composition designated HL-1491 XZP commercially available from H.B. Fuller Canada, Toronto, Ontario, Canada. The adhesive 58 may be applied to the garment-facing surface of the barrier layer 50 of the liquid-absorbing section 22 in various patterns, including complete adhesive coverage, parallel longitudinal lines, a line of adhesive following the perimeter of the structure, transverse lines of adhesive or the like.

Standard release paper 82 (shown only in Figure 3) covers the adhesive 58 before the napkin is used to prevent the unwanted adherence of the napkin to itself or foreign objets. The release paper is of conventional construction (*e.g.* silicone coated wet-laid Kraft wood pulp) and suitable papers are available from Tekkote Corporation (Leonia, New Jersey, USA), and bear the designation FRASER 30#/61629.

### The Liquid-Arresting Section

United with the liquid-absorbing section 22 is the liquid-arresting section 24. The liquid-arresting section 24 is a thin and flexible rear extension from the liquid-absorbing section 22, capable of conforming to the buttocks area of the wearer. The liquid-arresting section 24 appears as a natural extension of the liquid-absorbing section 22, and thus its longitudinal sides 72, 74 generally appear as extensions of the longitudinal sides 30, 32 of the liquid-absorbing section 22. Similarly the curvature at its transverse end 70 is similar to that of the first transverse side 26 of the liquid-absorbing section 22. (Such shape however is only preferred and is not essential to the present invention)

In the preferred embodiment shown in Figures 1 and 2, the liquid-arresting section 24 projects from the liquid-absorbing section 22 approximately 7.0 cm *(i.e.* its projection length 62 is approximately 7.0 cm). The projection length 62 of the liquid-arresting section 24 is the maximum distance that exists between the second transverse side 28 of the liquid-arresting section 22 and the transverse side 70 of the liquid-arresting section 22 (including the width of the seal 60, if any), measured along a line that is parallel to the longitudinal centerline 34 of the liquid-absorbing section 22. The liquid-arresting section 24 also has a width 76 of approximately 7.0 cm; the width 76 of the section 24 being the distance from one longitudinal side 72 of the section 24 to the other 74, measured along a line perpendicular to the line along which the projection length 62 is measured, through the midpoint thereof.

Referring to Figure 4, the liquid-arresting section 24 is a dual-layer structure comprising a cover layer part 54 that is continuous (and integrally-formed) with the cover layer 42 of the liquid-absorbing section 22. Both the cover layer part 54 and the cover layer 42 are formed of the same material. (Optionally, the cover layer part 54 of the liquid-arresting section 24 may contain a non-woven fabric to reduce what some users might perceive to be a hot sticky plastic feel; or it may be preferred to use a combination of a plastic cover with non-woven material in the liquid-arresting section 24.) The liquid-arresting section 24 also comprises a barrier layer part 56 that is continuous (and integrally-formed) with the barrier layer 50 of the liquid-absorbing section 22. Both the barrier layer part 56 and the barrier layer 50 are formed of the same material. The cover layer part 54 is secured to the barrier layer part 56 along a generally U-shaped peripheral seal line 60. Thus the peripheral seal 52 (in the second transverse end region of the liquid-absorbing section) and the seal line 60 define the liquid-arresting section 24) in the present embodiment.

No portion of the absorbent system 44 extends into the liquid-arresting section 24, nor are any additional independent absorbent materials present therein (except for the perhaps minimally absorbent cover layer part 54). The liquid-arresting section 24 is thus of reduced absorbent capacity and reduced caliper as compared with the liquid-absorbing section 22. The difference in caliper between the liquid-absorbing section 22 and the liquid-arresting section 24 that results from the absence of absorbent material in the liquid-arresting section 24 is clearly depicted in Figure 4. The ratio of average absorption capacity of the liquid-absorbing section 22 to average absorption capacity of the liquid-arresting layer 24 at least 2:1. Similarly, the ratio of caliper liquid-absorbing layer 22 to caliper of liquid-arresting layer 24 is at least 2:1. The ratio of the flexibility of the liquid-absorbing layer 22 to flexibility of the liquid-arresting layer 24 is at least 1:2. Exact values for each of these characteristics and ratios may be determined using the techniques described in the section entitled "Test Procedures".

Referring to Figure 3, the garment-facing surface of barrier layer part 56 of the liquid-arresting section 24 does not contain adhesive material 58 as does the garment-facing surface of the barrier layer 50 of the liquid-absorbing section 22. The liquid-arresting section 22 is therefore not adhered to the undergarment 66 when the napkin 20 is in use and is free to lift off and conform to the body of the wearer.

Each of the characteristics of the liquid-arresting section 24, *i.e*. its projection length 62 to cover at least a portion of a wearer's gluteal groove, its lack of adhesives on its garment-facing surface, its reduced caliper and its increased flexibility along a transverse axis, contributes to allow this part of the sanitary napkin 20 to better conform to the buttocks area. Such enhanced conformability improves the comfort potential of the product. In addition, the perception of comfort by the wearer is also likely to be improved because in practice users tend to associate comfort with reduced product thickness. Another advantage that results from these characteristics is the ability of the liquid-arresting section 24 to behave as a positioning system, maintaining the sanitary napkin 20 in place against the perineal region of the wearer. This feature is likely to further reduce the possibility of failures that may results from an improperly positioned sanitary napkin or having a sanitary napkin that shifts in position.

Contrary to what conventional wisdom dictates, a reduction of the absorption capacity in the rear portion of the sanitary napkin does not result in a higher failure incidence in that region. The present inventor has observed that most of the fluid discharge from the vaginal opening is captured by the liquid absorbing section 22, while only a small fraction may roll-off to the liquid-arresting section 24. In most cases, the absence of absorbing material in that region suffices to retain the menstrual liquid in place. This is due to the high surface tension developed between the liquid and the cover layer part 54. Thus, while the liquid is not absorbed inside the sanitary napkin as it would be when the liquid is discharged over the liquid-absorbing section 22, enough liquid retention exists in the liquid-arresting section 24 to keep the liquid in place and prevent if from soiling the undergarment of the wearer. At the same time, a number of benefits accrue from the gained flexibility and thickness reduction in the area of the liquid-arresting section 24.

Figure 5 illustrates in cross-section another embodiment of the present invention. This embodiment features a liquid-arresting section 24 containing a transfer layer part 64. The transfer layer part 64 is (although need not be) continuous with the transfer layer 46 of the liquid-absorbing section 22. The transfer layer part 64 of the liquid-arresting section 24 is substantially co-extensive with the cover layer part 54 and the barrier layer part 56. The only area of the liquid-arresting section 24 in which this co-extensive relationship is not maintained is the flange seal 60. One possible advantage of this embodiment is the provision of enhanced absorption capacity in the liquid-arresting section 24 that can be useful for applications where the sanitary napkin 20 is likely to be subjected to large volume input. The drawback, however, is an increase of the caliper and a reduction in the flexibility of the liquid-arresting section 24 that may have a negative impact on the comfort potential and the conformability of the sanitary napkin 20 to the anatomy of the user. Such negative impact is minimal in practice because the transfer layer part 64 is thin and sufficiently flexible.

Figure 6 illustrates in cross-section a further embodiment of the invention. This embodiment features a single layer absorbent system 44 having a tail element 68 that projects into the liquid-arresting section 24. The tail element 68 has a thickness that gradually diminishes toward the rear end of the sanitary napkin 20. This provides the napkin 20 with an absorption profile such that the absorption capacity diminishes along the longitudinal centerline 34 of the product when moving toward the rear end depending of course on the dimensions of the tail element 68 at any particular point.

Each of the above-described embodiments of the sanitary napkin 20 is fabricated in a conventional manner in accordance with conventional techniques. Specifically, a laminate structure, sometimes referred to in the art as a web, is created. This laminate structure comprises an expanse of the materials from which the napkin will be created. *I.e.* the laminate structure comprises an expanse of cover layer material overlying transfer layer material, which overlies absorbent layer material, all of which overlie an expanse of barrier layer material Some of the materials are necessarily not continuous within the laminate structure, and where such is the case, they are positioned precisely, one with respect to another, in the relationship they will occupy in the final products. The cover layer material and the barrier layer material are then bonded together by applying pressure in the appropriate positions, and what will become the peripheral seal is created. (The seal may also be made by means of heat-bonding, ultrasonic bonding, radio frequency sealing, mechanical crimping, and the like and combinations thereof.) The sealed structure is then severed by conventional means (*i.e.* die-cutting, fluid-jet cutting, or by laser) from the web to create a discrete article.

The adhesive material is then applied to the barrier layer in the appropriate positions, and release paper is applied to cover the adhesive. Alternatively, the adhesive, or the adhesive and the release paper may be applied to the web before the individual articles are severed therefrom. In use, the release paper is removed, and the napkin is positioned within the undergarment of a wearer.

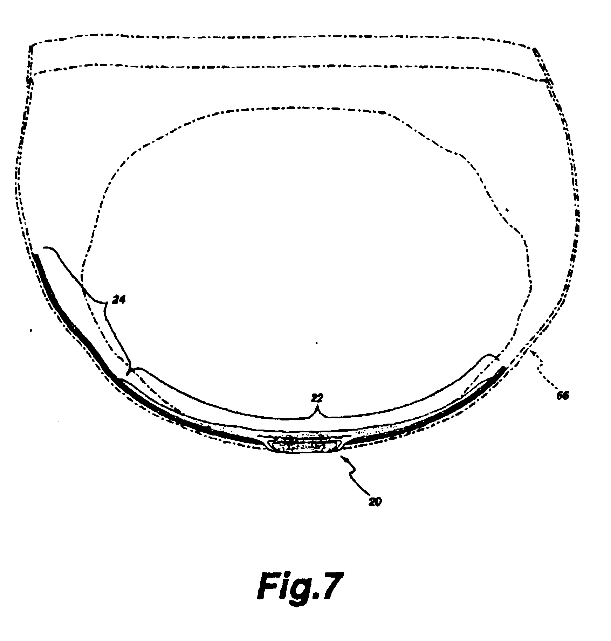

Figure 7 illustrates the sanitary napkin 20, positioned in an undergarment 66 shown in broken lines. The sanitary napkin 20 is depicted in the position it would attain when the undergarment 66 is in use, pulled such that its crotch portion presses against the perineal area of the user. In this position the liquid-absorbing section 22 of the sanitary napkin 20 covers at least in part the vaginal opening of the user. The liquid-arresting section 24 of the sanitary napkin 20 extends over at least a portion of the buttocks area. As mentioned earlier in this specification, the buttocks area is defined as the seat portion of the body that extends upwardly from the anal opening up to the lower rear portion. The liquid-arresting section 24 will confirm to the shape of the body of the wearer in the gluteal region, and will thus act to prevent rear end failures, particularly when the wearer is in a horizontal prone position. The liquid-arresting section 24 will also help to position the napkin as described above.

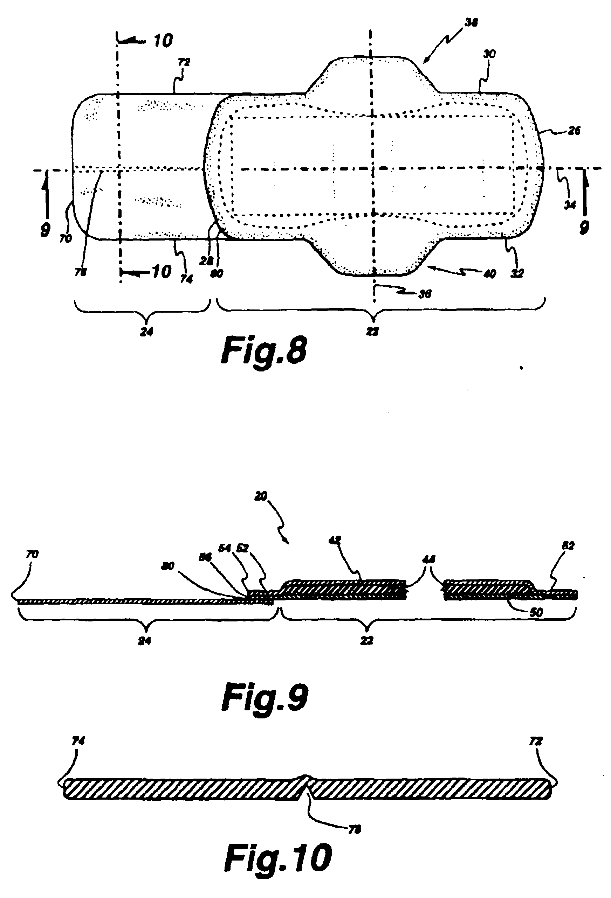

Another embodiment of the present invention is shown in Figure 8. In this embodiment the liquid-absorbing section 22 and the liquid-arresting section 24 are discrete components with a physical line of demarcation 80 between. The liquid-absorbing section 22 in this embodiment is generally similar to that described above in association with other embodiments, and is manufactured in a similar fashion. Referring to Figure 9, the liquid-arresting section 24, however simply comprises a single layer of the impervious material from which the barrier layer 50 is constructed. The liquid-arresting section 24 is independently cut from a web of material and is united by adhesive (or other conventional means) to the liquid-absorbing section 22 after that section's manufacture. The liquid-absorbing section 22 contains a preferential bending zone in the form of a score line 78. The score line 78 is co-linear with the longitudinal centerline 54 of the liquid-absorbing section 22. Referring to Figure 10, the score line 78 is such that when the napkin 20 is in use the liquid-arresting section 24 will preferentially bend inwards into the wearer's gluteal groove. This preferential bending will augment the ability of the section 24 to act as a positioning system, as described above.

### TEST PROCEDURES

### Caliper

The procedure consists of measuring the caliper of the material at 0.05 P.S.I. pressure with a compression meter using a 2 inch (5.08 cm) diameter foot (ASTM D-1777). In the case of the embodiment illustrated at figure 1, two measurements should suffice to establish the caliper ratio between the liquid-absorbing section 22 and the liquid-arresting section 24. Note that the measurements effected on the liquid-absorbing section 22 should be made inside the peripheral seal 52. For embodiments in which the thickness of a given section is not constant, several measurements should be made and the results averaged out. Such situation would arise in the case of the embodiment depicted in Figure 6 where the thickness of the liquid-arresting section 24 progressively diminishes toward the transverse end 70. A possible procedure is to divide the liquid-arresting section 24 in unit areas and measure the thickness of each unit are calculated and then average out the results. The average value will then constitute the caliper value associated with the section.

### Lateral flexibility/stability

The purpose of this test is to determine the lateral flexibility of an absorbent product or a portion of an absorbent product by subjecting the sample to side compression. The test procedure will be described with reference to Figure 6. The lateral flexibility of the sample is obtained by placing the sample in between two Plexiglas curves (simulating the inner thighs of the wearer). The stationary curve is attached to a compression load cell in an Instron Unit and a speed of 500 millimeters per minute is imparted to the moving curve. The sample is securely placed in between the two curves with three metal support rods stemming from the stationary curve and slidingly received in the moving curve. The force required to compress the sample to one-inch (2.54-cm) is recorded. In the case of the embodiments shown at Figures 4 and 5, the lateral flexibility is obtained by cutting each sample along the boundary separating the liquid-absorbing section 22 and the liquid-arresting section 24. Each sample is subjected to the testing procedure and the respective flexibility value is recorded. Note that each sample is placed in the instrument such that its longitudinal axis is perpendicular to the direction of movement of the moving curve.

### Absorption capacity

The average absorption capacity of the liquid-absorbing section and of the liquid-arresting section is established by measuring the total absorption capacity of each section by any standard procedure ( the test using a "GATS" measuring instrument is satisfactory) and dividing the total capacity value for that section by the surface area of the section to obtain an average capacity value expressed in terms of capacity per unit surface area.

Applications of the product and methods of the present invention for sanitary and other health-care uses can be accomplished by any sanitary protection, incontinence, medical and absorbent methods and techniques as are presently or prospectively known to those skilled in the art. Thus, it is intended that the present application cover the modifications and variations of this invention provided that they come within the scope of the appended claims and their equivalents.

## Claims

1. A sanitary napkin, comprising:
(A) a liquid-absorbing section for absorbing bodily exudate, said liquid-absorbing section having:
(i) two opposing longitudinal sides;
(ii) a first transverse side defining a front portion of said liquid-absorbing section;
(iii) a second transverse side opposing the first transverse side, the second transverse side defining a rear portion of said liquid-absorbing section;
(iv) an imaginary longitudinal centerline; and
(v) an imaginary transverse centerline;
said liquid-absorbing section comprising:
(i) a liquid-permeable cover layer, the cover layer having an external surface facing towards a wearer's body when the napkin is in use by the wearer,
(ii) a liquid-impervious barrier layer, the barrier layer having an external surface facing away from the wearer's body when the napkin is in use by the wearer,
(iii) an absorbent layer intermediate the cover layer and the barrier layer;
(iv) adhesive material on the external surface of the barrier layer, the adhesive material capable of releasably adhering the liquid-absorbing section to an undergarment of the wearer when the napkin is in use;
(B) a liquid-arresting section united with said liquid-absorbing section, said liquid-arresting section projecting rearward from the second transverse side of said liquid-absorbing section to a sufficient distance to at least partially cover a wearer's gluteal groove when the liquid-absorbing section is substantially aligned with the wearer's vaginal opening, the liquid-arresting section having an external surface facing away from the wearer's body when the article is in use by the wearer, the external surface being substantially free of adhesive; and
wherein: either said liquid-arresting section has a longitudinally extending preferential being zone, such that when the article is in use by the wearer, at least a portion of said liquid-arresting section is capable of bending at said preferential bending zone so as to enter the wearer's gluteal groove; or the liquid-absorbing section and said liquid-arresting section each have an average caliper, the average caliper of said liquid-arresting section being less than the average caliper of said liquid-absorbing section; or said liquid-absorbing section and said liquid-arresting section each have a flexibility, the flexibility of said liquid-arresting section being more than the flexibility of said liquid-absorbing section.

2. The sanitary napkin of claim 1, wherein said liquid-absorbing section and said liquid-arresting section each have an average absorption capacity, the average absorption capacity of said liquid-arresting section being less than the average absorption capacity of said liquid-absorbing section.

3. The sanitary napkin of claim 1 or claim 2, wherein said liquid-arresting section includes a liquid-impervious layer.

4. The sanitary napkin of claim 3, wherein the liquid-impervious layer of said liquid-arresting section is continuous with the barrier layer of said liquid-absorbing section.

5. The sanitary napkin of any one of claims 1 to 4, wherein said liquid-arresting section includes a liquid-permeable layer continuous with the cover layer of said liquid-absorbing section.

6. The sanitary napkin of claim 5, wherein said liquid-arresting section is substantially free of absorbent material between the liquid-permeable layer and the liquid-impervious layer.

7. The sanitary napkin of claim 5, wherein said liquid-arresting section includes absorbent material between the liquid-permeable layer and the liquid-impervious layer.

8. The sanitary napkin of claim 7, wherein the absorbent material is continuous with the absorbent layer of said liquid-absorbing section.

9. The sanitary napkin of claim 7 or claim 8, wherein said liquid-absorbing section further comprises a transfer layer and the absorbent material of said liquid-arresting section is continuous with the transfer layer.

10. The sanitary napkin of any one of claims 1 to 9, further comprising: a pair of flaps, one flap projecting laterally from each longitudinal side of said liquid-absorbent section, the flaps being capable of being folded over an edge of a crotch portion of an undergarment of the wearer, when the napkin is in use by the wearer.
